# EUROPEAN PATENT APPLICATION

(11) **EP 1 291 425 A2**
(43) Date of publication of application: **12.03.2003**
(21) Application number: 02255712.8
(22) Date of filing: 15.08.2002
(51) Int. Cl.: C12N 15/12, C07K 14/47, C07K 16/18, G01N 33/50, C12N 5/10

(54) **Human Na+/H+ exchanger protein and uses thereof**

(30) Priority: 31.08.2001 US 316675 P
(71) Applicant: Pfizer Products Inc., Groton, Connecticut 06340 (US)
(72) Inventor: Kennedy, Scott Paul, c/o Pfizer Global Res and Dev, Groton, Connecticut 06340 (US); Sun, Dexue, c/o Pfizer Global Research and Develp, Groton, Connecticut 06340 (US)
(74) Representative: England, Peter Michael

(57) **Abstract**

The invention is directed to two forms of isolated or purified human NHE2 polypeptide sequences, NHE2-LF and NHE2-SF, as well as the polynucleotide coding sequences that encode the human NHE2-LF and NHE2-SF polypeptides. Also featured are NHE2-LF- and NHE2-SF-related hosts, cell membrane preparations, vectors, and antibodies, as well as methods of using cells recombinantly expressing the human NHE2-LF or NHE2-SF, to identify agents that modulate human NHE activity.

SEQUENCE LISTING

<110>  Pfizer Products Inc.

<120>  HUMAN NHE2

<130>  PC11065ANIS

<140>  60/316675
<141>  2001-08-31

<150>  60/316675
<151>  2001-08-31

<160>  17

<170>  PatentIn version 3.1

<210>  1
<211>  2815
<212>  DNA
<213>  Homo sapiens

<400>  1
tactcactca ggctcgagcg gccgcccggg caggtctaga attcagcggc cgctgaattc      60
taggatgcgt tgagcgctcg gagggccaac cgccggtccc cttggcggca accggcggca     120
cccatggaac cactgggcaa ctggaggagc ctgcgggcgc cactgccccc gatgctgttg     180
ctgctgctcc tgcaggtggc ggggcccgtg ggcgccctgg cggagacctt gctgaacgcg     240
ccgagggcca tgggcaccag ttccagcccg cctagccctg cgagcgtggt ggctcccgga     300
acgacgctgt tcgaggagag ccggctgcct gtgtttacgc tggattaccc ccacgtgcag     360
atccccttcg agatcaccct ttggatcctg ctggcctccc tggccaagat tggcttccat     420
ctgtatcaca agttgcccac aatagtgcct gagagctgcc ttcttataat ggttggactt     480
ctactaggtg ggattatttt tggtgttgat gagaagtctc cccctgcaat gaagactgat     540

gtatttttct tgtacctcct cccacccatc gtgctggatg ccggctattt catgcccact 600
cgcccattct ttgagaacat tggcacgatt ttctggtatg ctgtggtagg gacactttgg 660
aattccattg gcattggggt gtctttgttt ggtatctgcc agatcgaagc attcggcctc 720
agcgacatca ctttgctcca gaacctgctc tttggcagct taatctcagc tgtcgatcct 780
gtggctgtgc ttgctgtctt tgagaacatt cacgtcaatg agcagctcta catcctggtc 840
tttggagagt ccctgctgaa tgatgcagta acagtggtcc tgtacaactt gttcaagtcg 900
ttttgccaga tgaaaaccat tgagaccatt gatgtgtttg caggaatcgc caacttcttt 960
gtagtgggga ttggcggggt gctgattggt atcttcttgg gctttatagc ggcatttact 1020
actcgattca cccataatat ccgagtgatc gagccactgt ttgttttcct gtacagttat 1080
ttgtcctaca tcacagctga aatgtttcac ctctcaggca tcatggcaat cactgcttgt 1140
gcaatgacta tgaataagta cgtagaagaa aatgtatctc agaaatccta cacgaccatc 1200
aagtacttca tgaagatgct gagcagtgtc agcgaaacct tgatcttcat cttcatgggt 1260
gtgtctaccg tgggcaagaa ccacgagtgg aactgggcct tcgtctgctt caccctggcc 1320
ttctgcctca tgtggcgagc cctgggtgtt tttgtcctga ctcaggtcat taataggttc 1380
cggaccattc ccctgacctt taaggaccag ttcatcattg cctatggagg acttcgaggt 1440
gccatctgtt ttgcgttagt gtttctcctt cctgctgctg tgtttcctcg gaaaaaattg 1500
tttattacgg ctgccattgt tgtcatattc tttactgtct tcattctggg aataactatt 1560
cgaccactgg tggagtttct tgatgtcaag aggtccaata agaaacaaca agctgtcagt 1620
gaagaaatct attgtcggtt gtttgatcat gtgaagactg gaattgaaga tgtttgtgga 1680
cattggggtc acaacttttg gagagacaag tttaagaagt ttgatgataa atatctgcgg 1740
aagcttttga ttcgggaaaa ccaaccaaag tcaagtattg tatctttata taaaaagctt 1800
gaaataaaac atgccattga gatggcagag actgggatga taagtactgt ccctacattt 1860
gcatctctaa atgattgtcg tgaagaaaaa ataaggaagg tcacgtccag tgaaactgat 1920
gaaattcgag aactcttatc aagaaatctc tatcaaatcc gtcagcgcac tttatcctac 1980
aacagacaca gtctgacagc agacacaagt gagaggcaag ccaaggagat tcggattcgc 2040
cggcgacaca gtttgcgaga aagcattagg aaggacagca gcttgaatcg agaacacagg 2100
gcttccactt caacctcccg atatttatcc ttacctaaaa atacgaagct tccagaaaag 2160
ctacaaaaga ggaggactat ttctattgca gatggcaata gcagcgactc agacgcagat 2220
gccgggacca ccgtgctcaa tttgcagccc agagccaggc gcttcttgcc agaacagttc 2280
tccaagaaat ccccccagtc ctataaaatg gaatggaaga atgaggtaga tgttgattct 2340
ggccgagata tgcccagcac ccccccaaca ccccacagca gagaaaaggg cacccagacg 2400
tcaggcttac tacagcagcc ccttctctct aaagaccagt ctggctcaga gagggaagac 2460
agtttgactg aaggcatccc gcccaagccg ccaccacggc tggtctggag ggcatcggaa 2520
cctggaagcc ggaaagcccg atttgggagt gagaagcctt aagagaagca gcgaaagcag 2580

atctgagtgt ctgacccagg acagctgtgg tttgtcactc tgaaacctga tgcaacagtg 2640
gaatccatgt aaaactctct gtgcatctaa atacttctgg agggcgacag attcatgcca 2700
cggataaatg aggcaaatcc gaagaaaagg aaaatcgaat aaaaaatagt cccacaaaat 2760
accttttgtg actaatgggt agcaatcgta ttatttgctg gcctgaagag aaaaa 2815

<210> 2
<211> 812
<212> PRT
<213> Homo sapiens

<400> 2

Met Glu Pro Leu Gly Asn Trp Arg Ser Leu Arg Ala Pro Leu Pro Pro
1               5                   10                  15

Met Leu Leu Leu Leu Leu Leu Gln Val Ala Gly Pro Val Gly Ala Leu
            20                  25                  30

Ala Glu Thr Leu Leu Asn Ala Pro Arg Ala Met Gly Thr Ser Ser Ser
        35                  40                  45

Pro Pro Ser Pro Ala Ser Val Val Ala Pro Gly Thr Thr Leu Phe Glu
    50                  55                  60

Glu Ser Arg Leu Pro Val Phe Thr Leu Asp Tyr Pro His Val Gln Ile
65                  70                  75                  80

Pro Phe Glu Ile Thr Leu Trp Ile Leu Leu Ala Ser Leu Ala Lys Ile
                85                  90                  95

Gly Phe His Leu Tyr His Lys Leu Pro Thr Ile Val Pro Glu Ser Cys
            100                 105                 110

Leu Leu Ile Met Val Gly Leu Leu Leu Gly Gly Ile Ile Phe Gly Val
        115                 120                 125

Asp Glu Lys Ser Pro Pro Ala Met Lys Thr Asp Val Phe Phe Leu Tyr
    130                 135                 140

Leu Leu Pro Pro Ile Val Leu Asp Ala Gly Tyr Phe Met Pro Thr Arg
145                 150                 155                 160

Pro Phe Phe Glu Asn Ile Gly Thr Ile Phe Trp Tyr Ala Val Val Gly
                165                 170                 175

Thr Leu Trp Asn Ser Ile Gly Ile Gly Val Ser Leu Phe Gly Ile Cys

            180                 185                 190

Gln Ile Glu Ala Phe Gly Leu Ser Asp Ile Thr Leu Leu Gln Asn Leu
        195                 200                 205

Leu Phe Gly Ser Leu Ile Ser Ala Val Asp Pro Val Ala Val Leu Ala
    210                 215                 220

Val Phe Glu Asn Ile His Val Asn Glu Gln Leu Tyr Ile Leu Val Phe
225                 230                 235                 240

Gly Glu Ser Leu Leu Asn Asp Ala Val Thr Val Val Leu Tyr Asn Leu
                245                 250                 255

Phe Lys Ser Phe Cys Gln Met Lys Thr Ile Glu Thr Ile Asp Val Phe
            260                 265                 270

Ala Gly Ile Ala Asn Phe Phe Val Val Gly Ile Gly Gly Val Leu Ile
        275                 280                 285

Gly Ile Phe Leu Gly Phe Ile Ala Ala Phe Thr Thr Arg Phe Thr His
    290                 295                 300

Asn Ile Arg Val Ile Glu Pro Leu Phe Val Phe Leu Tyr Ser Tyr Leu
305                 310                 315                 320

Ser Tyr Ile Thr Ala Glu Met Phe His Leu Ser Gly Ile Met Ala Ile
                325                 330                 335

Thr Ala Cys Ala Met Thr Met Asn Lys Tyr Val Glu Glu Asn Val Ser
            340                 345                 350

Gln Lys Ser Tyr Thr Thr Ile Lys Tyr Phe Met Lys Met Leu Ser Ser
        355                 360                 365

Val Ser Glu Thr Leu Ile Phe Ile Phe Met Gly Val Ser Thr Val Gly
    370                 375                 380

Lys Asn His Glu Trp Asn Trp Ala Phe Val Cys Phe Thr Leu Ala Phe
385                 390                 395                 400

Cys Leu Met Trp Arg Ala Leu Gly Val Phe Val Leu Thr Gln Val Ile
                405                 410                 415

Asn Arg Phe Arg Thr Ile Pro Leu Thr Phe Lys Asp Gln Phe Ile Ile
            420                 425                 430

Ala Tyr Gly Gly Leu Arg Gly Ala Ile Cys Phe Ala Leu Val Phe Leu
        435                 440                 445

Leu Pro Ala Ala Val Phe Pro Arg Lys Lys Leu Phe Ile Thr Ala Ala
450                 455                 460

Ile Val Val Ile Phe Phe Thr Val Phe Ile Leu Gly Ile Thr Ile Arg
465                 470                 475                 480

Pro Leu Val Glu Phe Leu Asp Val Lys Arg Ser Asn Lys Lys Gln Gln
                485                 490                 495

Ala Val Ser Glu Glu Ile Tyr Cys Arg Leu Phe Asp His Val Lys Thr
            500                 505                 510

Gly Ile Glu Asp Val Cys Gly His Trp Gly His Asn Phe Trp Arg Asp
        515                 520                 525

Lys Phe Lys Lys Phe Asp Asp Lys Tyr Leu Arg Lys Leu Leu Ile Arg
    530                 535                 540

Glu Asn Gln Pro Lys Ser Ser Ile Val Ser Leu Tyr Lys Lys Leu Glu
545                 550                 555                 560

Ile Lys His Ala Ile Glu Met Ala Glu Thr Gly Met Ile Ser Thr Val
                565                 570                 575

Pro Thr Phe Ala Ser Leu Asn Asp Cys Arg Glu Glu Lys Ile Arg Lys
            580                 585                 590

Val Thr Ser Ser Glu Thr Asp Glu Ile Arg Glu Leu Leu Ser Arg Asn
        595                 600                 605

Leu Tyr Gln Ile Arg Gln Arg Thr Leu Ser Tyr Asn Arg His Ser Leu
    610                 615                 620

Thr Ala Asp Thr Ser Glu Arg Gln Ala Lys Glu Ile Arg Ile Arg Arg
625                 630                 635                 640

Arg His Ser Leu Arg Glu Ser Ile Arg Lys Asp Ser Ser Leu Asn Arg
                645                 650                 655

Glu His Arg Ala Ser Thr Ser Thr Ser Arg Tyr Leu Ser Leu Pro Lys
            660                 665                 670

Asn Thr Lys Leu Pro Glu Lys Leu Gln Lys Arg Arg Thr Ile Ser Ile
        675                 680                 685

Ala Asp Gly Asn Ser Ser Asp Ser Asp Ala Asp Ala Gly Thr Thr Val
    690                 695                 700

Leu Asn Leu Gln Pro Arg Ala Arg Arg Phe Leu Pro Glu Gln Phe Ser
705                 710                 715                 720

Lys Lys Ser Pro Gln Ser Tyr Lys Met Glu Trp Lys Asn Glu Val Asp
                725                 730                 735

Val Asp Ser Gly Arg Asp Met Pro Ser Thr Pro Pro Thr Pro His Ser
            740                 745                 750

Arg Glu Lys Gly Thr Gln Thr Ser Gly Leu Leu Gln Gln Pro Leu Leu
        755                 760                 765

Ser Lys Asp Gln Ser Gly Ser Glu Arg Glu Asp Ser Leu Thr Glu Gly
    770                 775                 780

Ile Pro Pro Lys Pro Pro Pro Arg Leu Val Trp Arg Ala Ser Glu Pro
785                 790                 795                 800

Gly Ser Arg Lys Ala Arg Phe Gly Ser Glu Lys Pro
                805                 810

<210> 3

<211> 2836

<212> DNA

<213> Homo sapiens

<220>

<221> misc_feature

<222> (1)..(2836)

<223> n=a or g or c or t/u, unknown, or other

<400> 3
caagactcca tctcaaaaaa aaataataat aataataata aaattgtgtt attccatttc 60

cacagttcca gccctgaagg aaatctcaac ggggatttag aaaaaataaa atctgtacaa 120

tgtagataat gtgtacctat agcactccaa ttttaatgta acattaattt tttgtctaca 180

tgtgcttcat gaatagtttt aagggaaacc tctggtttgc actgtaatct gggaagccat 240

tgtaaagaca gtgatactga cttgggagat ggtgaaacag gacccactgg ttctatcaaa 300

gggaaccaat ttcctgtctc ccaaattcct ccataaccca agttgtgtgt ttttattttt 360

tccttaccag gcttccatct gtatcacaag ttgcccacaa tagtgcctga gagctgcctt 420

cttataatgg ttggacttct actaggtggg attatttttg gtgttgatga gaagtctccc 480

cctgcaatga agactgatgt atttttcttg tacctcctcc cacccatcgt gctggatgcc 540

ggctatttca tgcccactcg cccattcttt gagaacattg gcacgatttt ctggtatgct 600

gtggtaggga cactttggaa ttccattggc attggggtgt ctttgtttgg tatctgccag 660

atcgaagcat tcggcctcag cgacatcact ttgctccaga acctgctctt tggcagctta 720
atctcagctg tcgatcctgt ggctgtgctt gctgtctttg agaacattca cgtcaatgag 780
cagctctaca tcctggtctt tggagagtcc ctgctgaatg atgcagtaac agtggtcctg 840
tacaacttgt tcaagtcgtt ttgccagatg aaaaccattg agaccattga tgtgtttgca 900
ggaatcgcca acttctttgt agtggggatt ggcggggtgc tgattggtat cttcttgggc 960
tttatagcgg catttactac tcgattcacc cataatatcc gagtgatcga gccactgttt 1020
gttttcctgt acagttattt gtcctacatc acagctgaaa tgtttcacct ctcaggcatc 1080
atggcaatca ctgcttgtgc aatgactatg aataagtacg tagaagaaaa tgtatctcag 1140
aaatcctaca cgaccatcaa gtacttcatg aagatgctga gcagtgtcag cgaaaccttg 1200
atcttcatct tcatgggtgt gtctaccgtg ggcaagaacc acgagtggaa ctgggccttc 1260
gtctgcttca ccctggcctt ctgcctcatg tggcgagccc tgggtgtttt tgtcctgact 1320
caggtcatta ataggttccg gaccattccc ctgaccttta aggaccagtt catcattgcc 1380
tatggaggac ttcgaggtgc catctgtttt gcgttagtgt ttctccttcc tgctgctgtg 1440
tttcctcgga aaaaattgtt tattacggct gccattgttg tcatattctt tactgtcttc 1500
attctgggaa taactattcg accactggtg gagtttcttg atgtcaagag gtccaataag 1560
aaacaacaag ctgtcagtga agaaatctat tgtcggttgt ttgatcatgt gaagactgga 1620
attgaagatg tttgtggaca ttggggtcac aacttttgga gagacaagtt taagaagttt 1680
gatgataaat atctgcggaa gcttttgatt cgggaaaacc aaccaaagtc aagtattgta 1740
tctttatata aaaagcttga aataaaacat gccattgaga tggcagagac tgggatgata 1800
agtactgtcc ctacatttgc atctctaaat gattgtcgtg aagaaaaaat aaggaaggtc 1860
acgtccagtg aaactgatga aattcgagaa ctcttatcaa gaaatctcta tcaaatccgt 1920
cagcgcactt tatcctacaa cagacacagt ctgacagcag acacaagtga gaggcaagcc 1980
aaggagattc ggattcgccg gcgacacagt ttgcgagaaa gcattaggaa ggacagcagc 2040
ttgaatcgag aacacagggc ttccacttca acctcccgat atttatcctt acctaaaaat 2100
acgaagcttc cagaaaagct acaaaagagg aggactattt ctattgcaga tggcaatagc 2160
agcgactcag acgcagatgc cgggaccacc gtgctcaatt tgcagcccag agccaggcgc 2220
ttcttgccag aacagttctc caagaaatcc ccccagtcct ataaaatgga atggaagaat 2280
gaggtagatg ttgattctgg ccgagatatg cccagcaccc ccccaacacc ccacagcaga 2340
gaaaagggca cccagacgtc aggcttacta cagcagcccc ttctctctaa agaccagtct 2400
ggctcagaga gggaagacag tttgactgaa ggcatcccgc ccaagccgcc accacggctg 2460
gtctggaggg catcggaacc tggaagccgg aaagcccgat ttgggagtga gaagccttaa 2520
gagaagcagc gaaagcagat ctgagtgtct gacccaggac agctgtggtt tgtcactctg 2580
aaacctgatg caacagtgga atccatgtaa aactctctgt gcatctaaat acttctggag 2640

ggcgacagat tcatgccacg gataaatgag gcaaatccga agaaaaggaa aatcgaataa 2700
aaaatagtcc cacaaaatac cttttgtgac taatgggtag caatcgtatt atttgctggc 2760
ctgaagagaa aaannggtaa tncgtgcctt tnttgaactt gcaatgacag aacttgaaaa 2820
tnntaacant cactag 2836

<210> 4
<211> 697
<212> PRT
<213> Homo sapiens

<400> 4
Met Val Gly Leu Leu Leu Gly Gly Ile Ile Phe Gly Val Asp Glu Lys
1               5                   10                  15

Ser Pro Pro Ala Met Lys Thr Asp Val Phe Phe Leu Tyr Leu Leu Pro
            20                  25                  30

Pro Ile Val Leu Asp Ala Gly Tyr Phe Met Pro Thr Arg Pro Phe Phe
        35                  40                  45

Glu Asn Ile Gly Thr Ile Phe Trp Tyr Ala Val Val Gly Thr Leu Trp
    50                  55                  60

Asn Ser Ile Gly Ile Gly Val Ser Leu Phe Gly Ile Cys Gln Ile Glu
65                  70                  75                  80

Ala Phe Gly Leu Ser Asp Ile Thr Leu Leu Gln Asn Leu Leu Phe Gly
                85                  90                  95

Ser Leu Ile Ser Ala Val Asp Pro Val Ala Val Leu Ala Val Phe Glu
            100                 105                 110

Asn Ile His Val Asn Glu Gln Leu Tyr Ile Leu Val Phe Gly Glu Ser
        115                 120                 125

Leu Leu Asn Asp Ala Val Thr Val Val Leu Tyr Asn Leu Phe Lys Ser
    130                 135                 140

Phe Cys Gln Met Lys Thr Ile Glu Thr Ile Asp Val Phe Ala Gly Ile
145                 150                 155                 160

Ala Asn Phe Phe Val Val Gly Ile Gly Gly Val Leu Ile Gly Ile Phe
                165                 170                 175

Leu Gly Phe Ile Ala Ala Phe Thr Thr Arg Phe Thr His Asn Ile Arg
            180                 185                 190

Val Ile Glu Pro Leu Phe Val Phe Leu Tyr Ser Tyr Leu Ser Tyr Ile
        195                 200                 205

Thr Ala Glu Met Phe His Leu Ser Gly Ile Met Ala Ile Thr Ala Cys
    210                 215                 220

Ala Met Thr Met Asn Lys Tyr Val Glu Glu Asn Val Ser Gln Lys Ser
225                 230                 235                 240

Tyr Thr Thr Ile Lys Tyr Phe Met Lys Met Leu Ser Ser Val Ser Glu
                245                 250                 255

Thr Leu Ile Phe Ile Phe Met Gly Val Ser Thr Val Gly Lys Asn His
            260                 265                 270

Glu Trp Asn Trp Ala Phe Val Cys Phe Thr Leu Ala Phe Cys Leu Met
        275                 280                 285

Trp Arg Ala Leu Gly Val Phe Val Leu Thr Gln Val Ile Asn Arg Phe
    290                 295                 300

Arg Thr Ile Pro Leu Thr Phe Lys Asp Gln Phe Ile Ile Ala Tyr Gly
305                 310                 315                 320

Gly Leu Arg Gly Ala Ile Cys Phe Ala Leu Val Phe Leu Leu Pro Ala
                325                 330                 335

Ala Val Phe Pro Arg Lys Lys Leu Phe Ile Thr Ala Ala Ile Val Val
            340                 345                 350

Ile Phe Phe Thr Val Phe Ile Leu Gly Ile Thr Ile Arg Pro Leu Val
        355                 360                 365

Glu Phe Leu Asp Val Lys Arg Ser Asn Lys Lys Gln Gln Ala Val Ser
    370                 375                 380

Glu Glu Ile Tyr Cys Arg Leu Phe Asp His Val Lys Thr Gly Ile Glu
385                 390                 395                 400

Asp Val Cys Gly His Trp Gly His Asn Phe Trp Arg Asp Lys Phe Lys
                405                 410                 415

Lys Phe Asp Asp Lys Tyr Leu Arg Lys Leu Leu Ile Arg Glu Asn Gln
            420                 425                 430

Pro Lys Ser Ser Ile Val Ser Leu Tyr Lys Lys Leu Glu Ile Lys His
        435                 440                 445

Ala Ile Glu Met Ala Glu Thr Gly Met Ile Ser Thr Val Pro Thr Phe

450                 455                 460

Ala Ser Leu Asn Asp Cys Arg Glu Glu Lys Ile Arg Lys Val Thr Ser
465                 470                 475                 480

Ser Glu Thr Asp Glu Ile Arg Glu Leu Leu Ser Arg Asn Leu Tyr Gln
                485                 490                 495

Ile Arg Gln Arg Thr Leu Ser Tyr Asn Arg His Ser Leu Thr Ala Asp
            500                 505                 510

Thr Ser Glu Arg Gln Ala Lys Glu Ile Arg Ile Arg Arg Arg His Ser
        515                 520                 525

Leu Arg Glu Ser Ile Arg Lys Asp Ser Ser Leu Asn Arg Glu His Arg
    530                 535                 540

Ala Ser Thr Ser Thr Ser Arg Tyr Leu Ser Leu Pro Lys Asn Thr Lys
545                 550                 555                 560

Leu Pro Glu Lys Leu Gln Lys Arg Arg Thr Ile Ser Ile Ala Asp Gly
                565                 570                 575

Asn Ser Ser Asp Ser Asp Ala Asp Ala Gly Thr Thr Val Leu Asn Leu
            580                 585                 590

Gln Pro Arg Ala Arg Arg Phe Leu Pro Glu Gln Phe Ser Lys Lys Ser
        595                 600                 605

Pro Gln Ser Tyr Lys Met Glu Trp Lys Asn Glu Val Asp Val Asp Ser
    610                 615                 620

Gly Arg Asp Met Pro Ser Thr Pro Pro Thr Pro His Ser Arg Glu Lys
625                 630                 635                 640

Gly Thr Gln Thr Ser Gly Leu Leu Gln Gln Pro Leu Leu Ser Lys Asp
                645                 650                 655

Gln Ser Gly Ser Glu Arg Glu Asp Ser Leu Thr Glu Gly Ile Pro Pro
            660                 665                 670

Lys Pro Pro Pro Arg Leu Val Trp Arg Ala Ser Glu Pro Gly Ser Arg
        675                 680                 685

Lys Ala Arg Phe Gly Ser Glu Lys Pro
    690                 695

<210>  5

<211>  32

<212>  DNA

<213>  Homo sapiens


<400>  5
agacacaagt gagaggcaag ccaaggagat tc                                    32

<210>  6

<211>  19

<212>  DNA

<213>  Homo sapiens


<400>  6
ttaaattttc aagttctgt                                                   19

<210>  7

<211>  30

<212>  DNA

<213>  Homo sapiens


<400>  7
gtggggattg gcggggtgct gattggtatc                                       30

<210>  8

<211>  31

<212>  DNA

<213>  Homo sapiens


<400>  8
aaagtgcgct gacggatttg atagagattt c                                     31

<210>  9

<211>  30

<212>  DNA

<213>  Homo sapiens


<400>  9
gataccaatc agcaccccgc caatccccac                                       30

<210>  10

<211>  20
<212>  DNA
<213>  Homo sapiens

<400>  10
gtgccggaga gctgtcttct                                                    20

<210>  11
<211>  45
<212>  DNA
<213>  Homo sapiens

<400>  11
aagcatccgt cagcatcggc aggacaactt tttttttttt ttttt                        45

<210>  12
<211>  17
<212>  DNA
<213>  Homo sapiens

<400>  12
agcatcggca ggacaac                                                       17

<210>  13
<211>  19
<212>  DNA
<213>  Homo sapiens

<400>  13
gagacttctc aacaccaaa                                                     19

<210>  14
<211>  28
<212>  DNA
<213>  Homo sapiens

<400>  14
cccacctagt agaagtccaa ccattata                                           28

<210> 15

<211> 22

<212> DNA

<213> Homo sapiens

<400> 15
gtaatacgac tcactatagg gc 22

<210> 16

<211> 19

<212> DNA

<213> Homo sapiens

<400> 16
actatagggc acgcgtggt 19

<210> 17

<211> 35

<212> DNA

<213> Homo sapiens

<400> 17
ggcagccggc tctcctcgaa cagcgtcgtt ccggg 35

## Description

This application claims the benefit of U.S. Provisional Patent Application No. 60/316,675, filed August 31, 2001, incorporated in its entirety herein by reference.

### Field of the Invention

The present invention features human NHE2 polynucleotide and polypeptide sequences as well as assay methods for identifying agents that modulate NHE2 activity.

### Background

Na⁺/H⁺ exchangers (NHEs) are plasma membrane transport proteins that mediate electroneutral exchange of extracellular Na⁺ with H⁺ in a 1:1 stoichiometry. These exchangers have been implicated in many important cellular functions, including maintenance of intracellular pH, cell volume regulation, cell proliferation, and transcellular transport of Na⁺ (Aronson, Annu. Rev. Physiol. 47: 545-60, 1985; Grinstein and Rothstein, J. Membr. Biol. 90: 1-12, 1986; Grinstein et al., Biochim. Biophys. Acta 988: 73-97, 1989; Noel and Pouyssegur, Am. J. Physiol. (Cell Physiol.) 37: C283-96, 1995; Tse et al., J. Membr. Biol. 135: 93-108, 1993). Evidence suggests that modulating NHEs can play a role in treating renal acid-base disorders, essential hypertension, cancer, and tissue or organ hypertrophy (Mahnensmith and Aronson, Circ. Res. 56: 773-388,1985; Harguindey and Cragoe, Medical Hypothesis 39: 229-37, 1992).

Studies have identified a family of functionally and structurally related mammalian isoforms of NHE, termed NHEs 1-6 (Brant et al., Am. J. Physiol. 269: (Cell Physiol.) 38: C198-C206, 1995; Collins et al., Proc. Natl. Acad. Sci. USA 90: 3938-42, 1993; Klanke et al., Genomics 25: 615-22, 1995; Orlowski et al., J. Biol. Chem. 267: 9331-39, 1992; Sardet et al., Cell 56: 271-80, 1989; Szpirer et al., Mamm. Genome 5: 153-59, 1994; Tse et al., J. Biol. Chem. 267: 9340-46, 1992; Tse et al., J. Membr. Biol. 135: 93-108, 1993; and Wang et al., J. Biol. Chem. 268:11925-28, 1993).

### Summary of the Invention

The present invention features human NHE2 polynucleotide and polypeptide sequences as well as assay methods for identifying agents that modulate NHE2 activity. In a first aspect, the invention provides an isolated or purified polypeptide comprising the amino acid sequence of SEQ ID NO: 2 or 4.

In a second aspect, the invention features an isolated or purified polypeptide comprising an amino acid sequence encoded by a polynucleotide which will hybridize under highly stringent conditions with the complement of the coding sequence shown in SEQ ID NO: 1 or 3; or an amino acid sequence having at least 95% identity to an amino acid sequence comprising SEQ ID NO: 2 or 4; wherein the polypeptide has Na⁺/H⁺ exchange (NHE) activity.

In a related aspect, the invention provides an isolated or purified polynucleotide comprising a nucleic acid sequence encoding SEQ ID NO: 2 or SEQ ID NO: 4; or the coding sequence of SEQ ID NO: 1 or 3. Preferably, the polynucleotide comprises the coding sequence of SEQ ID NO: 1 or SEQ ID NO: 3. The invention also features a vector comprising this polynucleotide.

Another aspect of the invention features an isolated or purified polynucleotide comprising a nucleic acid sequence which hybridizes with the complement of the full length coding sequence of SEQ ID NO: 1 or 3 under highly stringent conditions, wherein the polynucleotide encodes a polypeptide with NHE activity. The invention also features a vector comprising this polynucleotide.

Related aspects feature an antibody that selectively binds to a polypeptide, and a host expressing a heterologous polypeptide, or a cell membrane derived thereof, wherein the polypeptide comprises the amino acid sequence of SEQ ID NO: 2 or 4, or comprises an amino acid sequence encoded by a polynucleotide which will hybridize under highly stringent conditions with the complement of the coding sequence shown in SEQ ID NO: 1 or 3, or comprises an amino acid sequence having at least 95% identity to an amino acid sequence comprising SEQ ID NO: 2 or SEQ ID NO: 4; and wherein the polypeptide has Na⁺/H⁺ exchange (NHE) activity.

Also featured by the invention is a method of screening for an agent that modulates human NHE2 activity comprising contacting an agent with a host cell that expresses a heterologous NHE2 comprising SEQ ID NO: 2 or 4, and measuring NHE activity in the cell, wherein a difference between the NHE activity in the presence of the agent and in the absence of the agent is indicative that the agent modulates NHE activity. Preferably, the heterologous NHE2 comprises SEQ ID NO: 2 or SEQ ID NO: 4, the host cell lacks endogenous NHE activity, and/or the host cell is a PS120 cell.

Those skilled in the art will fully understand the terms used herein in the description and the appendant claims to describe the present invention. Nonetheless, unless otherwise provided herein, the following terms are as described immediately below.

An "agent that increases NHE2 activity" refers to a molecule which intensifies or mimics the biological activity of a NHE2 polypeptide. Such agents (i.e., agonists) may include proteins, nucleic acids, carbohydrates, small molecules, or any other compound or composition which increases the activity of a NHE2 either by increasing the amount of NHE2 present in a cell or by increasing the catalytic activity of a NHE2 polypeptide.

An "agent that decreases NHE2 activity" refers to a molecule which inhibits or attenuates the biological activity of a NHE2 polypeptide. Such agents (i.e., antagonists) may include proteins such as anti-NHE2 antibodies, nucleic acids, carbohydrates, small molecules, or any other compound or composition which decreases the activity of a NHE2 polypeptide either by reducing the amount of NHE2 polypeptide present in a cell, or by decreasing the catalytic activity of a NHE2 polypeptide.

An "allelic variant" is an alternative form of the gene encoding a NHE2 polypeptide. Allelic variants may result from at least one mutation in the nucleic acid sequence and may result in altered mRNAs or in polypeptides whose structure or function may or may not be altered. A gene may have none, one, or many allelic variants of its naturally occurring form. Common mutational changes which give rise to allelic variants are generally ascribed to naturally-occurring deletions, additions, or substitutions of nucleotides. Each of these types of changes may occur alone, or in combination with the others, one or more times in a given sequence.

An "altered" nucleic acid sequence encoding a NHE2 polypeptide includes a sequence with a deletion, insertion, or substitution of different nucleotides, resulting in a polypeptide with at least one functional characteristic of a NHE2 polypeptide. Included within this definition are polymorphisms which may or may not be readily detectable using a particular oligonucleotide probe of the polynucleotide encoding a NHE2 polypeptide. The encoded protein may also be "altered," and may contain one or more deletions, insertions, or substitutions of amino acid residues which produce a silent change and result in a NHE2 polypeptide that is substantially equivalent functionally to a known NHE2 polypeptide. Deliberate amino acid substitutions may be made on the basis of similarity in polarity, charge, solubility, hydrophobicity, hydrophilicity, and/or the amphipathic nature of the residues, as long as NHE2 polypeptide is substantially functionally equivalent, e.g., in catalytic or immunologic activity.

"Amplification" relates to the production of additional copies of a nucleic acid sequence. It is generally carried out using polymerase chain reaction (PCR) technologies well known in the art.

A "composition" comprising a given polynucleotide or polypeptide may comprise a dry formulation or an aqueous solution.

"Conservative amino acid substitutions" are those substitutions that, when made, least interfere with the properties of the original protein, i.e., the structure and especially the function of the protein is conserved and not significantly changed by such substitutions. Examples of conservative amino acid substitutions include the following: Ala replaced with Gly or Ser; Arg replaced with His or Lys; Asn replaced with Asp, Gln, or His; Asp replaced with Asn or Glu; Cys replaced with Ala or Ser; Gln replaced with Asn, Glu, or His; Glu replaced with Asp, Gln, or His; Gly replaced with Ala; His replaced with Asn, Arg, Gln, or Glu; lle replaced with Leu or Val; Leu replaced with lle or Val; Lys replaced with Arg, Gln, or Glu; Met replaced with Leu or lie; Phe replaced with His, Met, Leu, Trp, or Tyr; Ser replaced with Cys or Thr; Thr replaced with Ser or Val; Trp replaced with Phe or Tyr; Tyr replaced with His, Phe, or Trp; and Val replaced with lle, Leu, or Thr. Conservative amino acid substitutions generally maintain the same, or essentially the same (a) structure of the polypeptide backbone in the area of the substitution, for example, as a beta sheet or alpha helical conformation, (b) charge or hydrophobicity of the molecule at the site of the substitution, and/or (c) bulk of the side chain.

The term "derivative" refers to the chemical modification of a polypeptide or polynucleotide sequence. Chemical modifications of a polynucleotide sequence can include, for example, replacement of hydrogen by an alkyl, acyl, hydroxyl, or amino group. A derivative polynucleotide encodes a polypeptide which retains at least one biological or immunological function of the natural molecule. A derivative polypeptide is one modified by glycosylation, pegylation, or any other process that retains at least one biological or immunological function of the polypeptide from which it was derived.

A "fragment" is a unique portion of a NHE2 polypeptide or the polynucleotide encoding a NHE2 polypeptide which is identical in sequence to, but shorter in length than, the parent sequence. A fragment used as a probe, primer, antigen, therapeutic molecule, or for other purposes, may be at least 5, 10, 15, 20, 25, 30, 40, 50, 60, 75, 100, 150, 250, or at least 500 contiguous nucleotides or amino acid residues in length. Fragments may be preferentially selected from, or lack, certain regions of a molecule.

The term "identity" refers to a degree of complementarity. There may be partial similarity or complete identity. The word "similarity" may substitute for the word "identity." A partially complementary sequence that at least partially inhibits an identical sequence from hybridizing to a target nucleic acid is referred to as "substantially similar." The inhibition of hybridization of the completely complementary sequence to the target sequence may be examined using a hybridization assay (Southern or Northern blot, solution hybridization, and the like) under conditions of reduced stringency. A substantially similar sequence or hybridization probe will compete for and inhibit the binding of a completely similar (identical) sequence to the target sequence under conditions of reduced stringency. This is not to say that conditions of reduced stringency are such that non-specific binding is permitted. Rather, reduced stringency conditions require that the binding of two sequences to one another be a specific (i.e., a selective) interaction. The absence of non-specific binding may be tested by the use of a second target sequence which lacks even a partial degree of complementarity (e.g., less than about 30% similarity or identity). In the absence of non-specific binding, the substantially similar sequence or probe will not hybridize to the second non-complementary target sequence.

The phrases "percent identity" and "% identity," as applied to polynucleotide sequences, refer to the percentage of residue matches between at least two polynucleotide sequences aligned using a standardized algorithm. Such an algorithm may insert, in a standardized and reproducible way, gaps in the sequences being compared in order to optimize alignment between two sequences, and therefore achieve a more meaningful comparison of the two sequences. Percent identity between polynucleotide sequences may be determined using the default parameters of the CLUSTAL V algorithm as incorporated into the MegAlign® version 3.12e sequence alignment program. This program is part of the LASERGENE software package, a suite of molecular biological analysis programs (DNASTAR, Madison, Wl). CLUSTAL V is described in Higgins and Sharp, CABIOS 5:151-153, 1989, and in Higgins et al., CABIOS 8:189-19, 1992. Percent identity is reported by CLUSTAL V as the "percent similarity" between aligned polynucleotide sequence pairs. Alternatively, a suite of commonly used and freely available sequence comparison algorithms is provided by the National Center for Biotechnology Information (NCBI) Basic Local Alignment Search Tool (BLAST) (Altschul et al., J. Mol. Biol. 215:403- 410, 1990), which is available from several sources, including the NCBI, Bethesda, MD, and at http://www.ncbi.nim.nih.gov/blast/. The BLAST software suite includes various sequence analysis programs including "blastn," that are used to align a known polynucleotide sequence with other polynucleotide sequences from a variety of databases. Also available is a tool called "BLAST 2 Sequences" that is used for direct pairwise comparison of two nucleotide sequences. "BLAST 2 Sequences" can be accessed and used interactively at http:/www.ncbi.nlm.nih.gov/blast/bl2seq/bl2.html. The "BLAST 2 Sequences" tool can be used for both blastn and blastp (discussed below). BLAST programs are commonly used with gap and other parameters set to default settings. For example, to compare two nucleotide sequences, one may use blastn with the "BLAST 2 Sequences" tool Version 2.0.9 (May-07-1999) using either Blossum 62 matrix or PAM250 matrix, a gap weight of 40, 50, 60, 70, or 80, and a length weight of 1, 2, 3, 4, 5, or 6. Percent identity may be measured over the length of an entire defined sequence, for example, as defined by a particular SEQ ID number, or may be measured over a shorter length, for example, over the length of a fragment taken from a larger, defined sequence, for instance, a fragment of at least 20, at least 30, at least 40, at least 50, at least 70, at least 100, or at least 200 contiguous nucleotides. Such lengths are exemplary only, and it is understood that any fragment length disclosed by the sequences shown herein may be used to describe a length over which percentage identity may be measured. Nucleic acid sequences that do not show a high degree of identity may nevertheless encode similar amino acid sequences due to the degeneracy of the genetic code. It is understood that changes in a nucleic acid sequence can be made using this degeneracy to produce multiple nucleic acid sequences encompassed by the invention that all encode the same or substantially the same NHE2 polypeptide.

The phrases "percent identity" and "% identity," as applied to polypeptide sequences, refer to the percentage of residue matches between at least two polypeptide sequences aligned using a standardized algorithm. Methods of polypeptide sequence alignment are well known. Some alignment methods take into account conservative amino acid substitutions. Such conservative substitutions, explained in more detail above, generally preserve the hydrophobicity and acidity at the site of substitution, thus preserving the structure and function of the polypeptide. Percent identity between polypeptide sequences may be determined using the default parameters of the CLUSTAL V algorithm as incorporated into the MegAlign® sequence alignment program (DNASTAR, Madison, WI). The PAM250 matrix is selected as the default residue weight table. As with polynucleotide alignments, the percent identity is reported by CLUSTAL V as the "percent similarity" between aligned polypeptide sequence pairs.

Alternatively, the NCBI BLAST software suite may be used. For example, for a pairwise comparison of two polypeptide sequences, one may use the "BLAST 2 Sequences" tool Version 2.0.9 (May-07-1999) with blastp set at default parameters. Such default parameters may be, for example, using Blossum 62 Matrix, score = 50, and word length = 3. Percent identity may be measured over the length of an entire defined polypeptide sequence, for example, as defined by a particular SEQ ID number, or may be measured over a shorter length, for example, over the length of a fragment taken from a larger, defined polypeptide sequence, for instance, a fragment of at least 15, at least 20, at least 30, at least 40, at least 50, at least 70, or at least 100 contiguous residues. Such lengths are exemplary only, and it is understood that any fragment length supported by the sequences shown herein, including the Figures and Sequence Listing, may be used to describe a length over which percentage identity may be measured.

By a "host" is meant a transgenic cell (e.g., mammalian, bacterial, insect) or an animal (e.g., non-human mammal) that is transfected with, and capable of expressing, a heterologous polynucleotide.

A "heterologous" polynucleotide is one which is foreign, or non-naturally occurring, or non-naturally positioned in the genome of the host cell.

"Hybridization" refers to the process by which a polynucleotide strand anneals with a complementary strand through base pairing under defined hybridization conditions. Specific hybridization is an indication that two nucleic acid sequences share a high degree of identity. Specific hybridization complexes form under permissive annealing conditions and remain hybridized after the "washing" step(s). The washing step(s) is (are) particularly important in determining the stringency of the hybridization process, with more stringent conditions allowing less non-specific binding, i.e., binding between pairs of nucleic acid strands that are not perfectly matched. Permissive conditions for annealing of nucleic acid sequences are routinely determinable by one of ordinary skill in the art and may be consistent among hybridization experiments, whereas wash conditions may be varied among experiments to achieve the desired stringency, and therefore hybridization specificity. Permissive annealing conditions occur, for example, at 68°C in the presence of about 6X SSC, about 1% (w/v) SDS, and about 100 pg/ml denatured salmon sperm DNA.

Generally, stringency of hybridization is expressed, in part, with reference to the temperature under which the wash step is carried out. Generally, such wash temperatures are selected to be about 5°C to 20°C lower than the thermal melting point (Tm) for the specific sequence at a defined ionic strength and pH. The Tm is the temperature (under defined ionic strength and pH) at which 50% of the target sequence hybridizes to a perfectly matched probe. An equation for calculating Tm and conditions for nucleic acid hybridization are well known and can be found in Sambrook et al., 1989, *Molecular Cloning: A Laboratory Manual*, 2^{nd} ed., Vol. 1-3, Cold Spring Harbor Press, Plainview, NY; specifically see Vol. 2, chapter 9.

High stringency conditions for hybridization between polynucleotides of the present invention include wash conditions of about 55-68°C in the presence of about 0.2-1.0X SSC and about 0.1% SDS, for about 1 hour.

In general, hybridization reactions can be carried out at temperatures of about 65°C, 60°C, 55°C, or 42°C. SSC concentration may be varied from about 0.1 to 2X SSC, with SDS being present at about 0.1%. Typically, blocking reagents are used to block non-specific hybridization. Such blocking reagents include, for instance, denatured salmon sperm DNA at about 100-200 pg/ml. Organic solvent, such as formamide at a concentration of about 35-50% v/v, may also be used under particular circumstances, such as for RNA:DNA hybridizations. Useful variations on these wash conditions will be readily apparent to those of ordinary skill in the art. Hybridization, particularly under high stringency conditions, is suggestive of evolutionary similarity between the nucleotides, which is strongly indicative of a similar role for the nucleotides and their encoded polypeptides.

The term "hybridization complex" refers to a complex formed between two nucleic acid sequences by virtue of the formation of hydrogen bonds between complementary bases. A hybridization complex may be formed between sequences present in solution or formed between one nucleic acid sequence present in solution and another nucleic acid sequence immobilized on a solid support (e.g., paper, membranes, filters, chips, pins or glass slides).

By "isolated or purified" is meant changed from the natural state "by the hand of man." If a polynucleotide or polypeptide exists in nature, then it is "isolated or purified" if it is changed and/or removed from its original environment. For example, an "isolated or purified" polynucleotide is separated from other polynucleotides with which it is associated in nature. For example, a cDNA sequence that is removed from intronic sequence normally associated with the coding sequence is "isolated or purified." An "isolated or purified" polynucleotide sequence may be introduced into host cells in culture or in whole organisms for transient or stable expression and still be "isolated and purified," because the polynucleotide would not be in its naturally occurring form or environment. However, polynucleotide sequences as members of cDNA libraries are excluded from what is meant by "isolated or purified." An "isolated or purified" polypeptide is separated from at least one cellular component with which it is associated in nature. Preferably, the polypeptide is at least 60% free, more preferably, at least 75% free, and, most preferably, at least 90% free from other components.

By "modulates" is meant increases or decreases (including a complete elimination).

"Operably linked" refers to the situation in which a first nucleic acid sequence is placed in a functional relationship with a second nucleic acid sequence. For example, a promoter is operably linked to a coding sequence if the promoter functions to regulate transcription of the coding sequence. Generally, operably linked DNA sequences may be in close proximity or contiguous and, where necessary to join two protein coding regions, in the same reading frame.

"Polynucleotide" generally refers to any RNA (e.g., mRNA), RNA-like, DNA (e.g., cDNA or genomic), or DNA like sequences, including, without limit, single-stranded, double-stranded, and triple-stranded sequences, sense or antisense strands, sequences generated using nucleotide analogs, hybrid molecules comprising RNA and DNA, and RNA or DNA containing modified bases. The polynucleotide can be naturally-occurring or synthesized.

The term "polypeptide" refers to an amino acid sequence, oligopeptide, peptide, polypeptide, or protein sequence, or a fragment of any of these, and to naturally occurring or synthetic molecules. It includes amino acid sequences modified either by natural processes, such as post-translational processing, or by chemical modifications well known in the art (see, e.g., *Proteins - Structure and Molecular Properties*, Ed. Creighton, W.H. Freeman and Co., New York, NY, 2^{nd} Ed, 1993; *Posttranslational Covalent Modification of Proteins*, Ed. Johnson, Academic Press, New York, NY, 1983; Seifter et al., Meth. Enzymol., 182: 626-46, 1990; and Rattan et al., Ann. NY Acad. Sci. 663: 48-62, 1992). Known modifications include, but are not limited to, acetylation, acylation, ADP-ribosylation, amidation, covalent attachment of flavin, heme moiety covalent attachment, covalent attachment of a nucleotide or nucleotide derivative, lipid or lipid derivative, or phosphotidylinositol, cross linking, cyclization, disulfide bond formation, demethylation, formation of cystine or pyroglutamate, formylation, gamma carboxylation, glycosylation, GPI anchor formation, hydroxylation, iodination, methylation, myristoylation, oxidation, proteolytic processing, phosphorylation, prenylation, racemization, selenoylation, sulfation, transfer RNA-mediated addition of amino acids to proteins, such as arginylation and ubiquitination.

By "NHE2 activity" is meant NHE2-mediated Na⁺/H⁺ exchange *in vitro, in vivo*, or *in situ*.

A "substitution" refers to the replacement of one or more amino acids or nucleotides by different amino acids or nucleotides, respectively.

"Transformation" or "transfection" describes a process of genetic modification by which heterologous DNA enters and renders a recipient cell capable of expressing the heterologous DNA. Transformation may occur in a prokaryotic or eukaryotic host cell according to various methods well known in the art. The method is selected based on the type of host cell being transformed and includes, but is not limited to, viral infection, electroporation, heat shock, lipofection, and particle bombardment. The terms "transformed cells" or "transfected cells" include stably transformed cells in which the inserted DNA is capable of replication either as an autonomously replicating plasmid or as part of the host chromosome, as well as transiently transformed or transfected cells which express the inserted DNA or RNA for limited periods of time. All of such transformed or transfected cells are referred to as "transgenic."

A "variant" of a particular nucleic acid sequence is defined as a nucleic acid sequence having at least 40% sequence identity to the particular nucleic acid sequence over a certain length of one of the nucleic acid sequences using blastn with the "BLAST 2 Sequences" tool Version 2.0.9, set at default parameters. Such sequences may show, for example, at least 50%, at least 60%, at least 70%, at least 80%, at least 85%, at least 90%, at least 95%, or at least 98%, or greater, sequence identity over a certain defined length. A variant may be described as, for example, an "allelic" (as defined above), "splice," "species," or "polymorphic" variant. A splice variant may have significant identity to a reference molecule, but will generally have a greater or lesser number of polynucleotides due to alternate splicing of exons during mRNA processing. The corresponding polypeptide may possess additional functional domains or lack domains that are present in the reference molecule. Species variants are polynucleotide sequences that vary from one species to another. The resulting polypeptides generally will have significant amino acid identity relative to each other. A polymorphic variant is a variation in the polynucleotide sequence of a particular gene between individuals of a given species. Polymorphic variants also may encompass "single nucleotide polymorphisms" (SNPs) in which the polynucleotide sequence varies by one nucleotide base. The presence of SNPs may be indicative of, for example, a certain population, a disease state, or a propensity for a disease state.

Other features and advantages of the invention will be apparent from the following detailed description and from the claims. While the invention is described in connection with specific embodiments, it will be understood that other changes and modifications that may be practiced are also part of this invention and are also within the scope of the appendant claims. Additional guidance with respect to making and using nucleic acids and polypeptides is found in standard textbooks of molecular biology, protein science, and immunology (see, e.g., Davis et al., *Basic Methods in Molecular Biology*, Elsevir Sciences Publishing, Inc., New York, NY,1986; Hames et al., *Nucleic Acid Hybridization,* IL Press, 1985; *Molecular Cloning,* Sambrook et al., *Current Protocols in Molecular Biology,* Eds. Ausubel et al., John Wiley and Sons; *Current* Protocols *in Human Genetics*, Eds. Dracopoli et al., John Wiley and Sons; *Current Protocols in Protein Science*, Eds. John E. Coligan et al., John Wiley and Sons; and *Current Protocols in Immunology*, Eds. John E. Coligan et al., John Wiley and Sons). All publications mentioned herein are incorporated by reference in their entireties.

### Description of the Figures

Fig. 1 shows the polynucleotide sequence of human NHE2-Long Form (NHE2-LF) (SEQ ID NO: 1).
Fig. 2 shows the predicted amino acid sequence of human NHE2-LF (SEQ ID NO: 2).
Fig. 3 shows the polynucleotide sequence of human NHE2-Short Form (NHE2-SF) (SEQ ID NO: 3).
Fig. 4 shows the predicted amino acid sequence of human NHE2-SF (SEQ ID NO: 4).

### Detailed Description

### The Nucleotide Coding Sequence and Amino Acid Sequence for Human NHE2

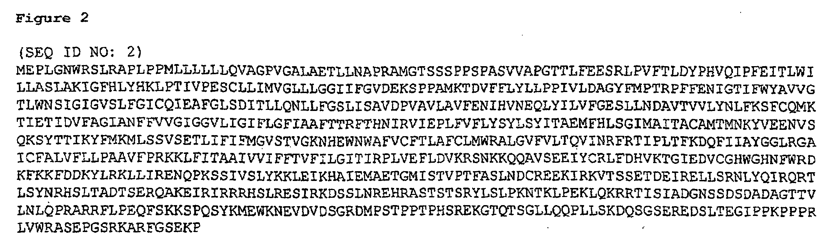

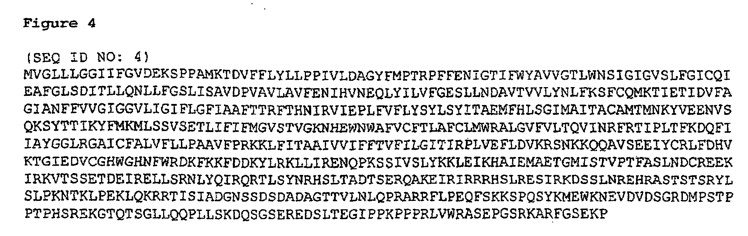

The invention encompasses the two forms of isolated or purified human NHE2 polypeptide sequences, NHE2-LF and NHE2-SF, for example, as shown in Fig. 2 (SEQ ID NO: 2), and in Fig. 4 (SEQ ID NO: 4), respectively.

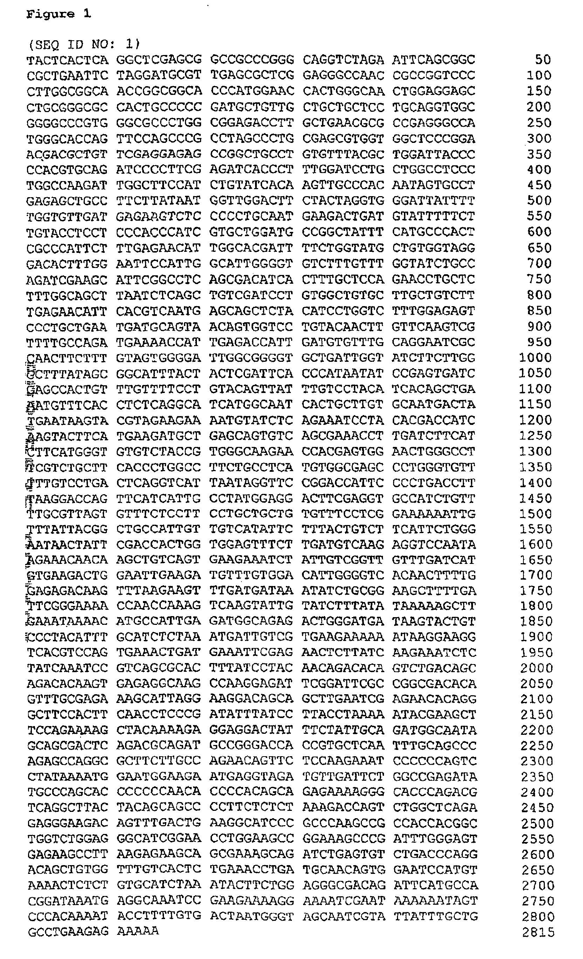

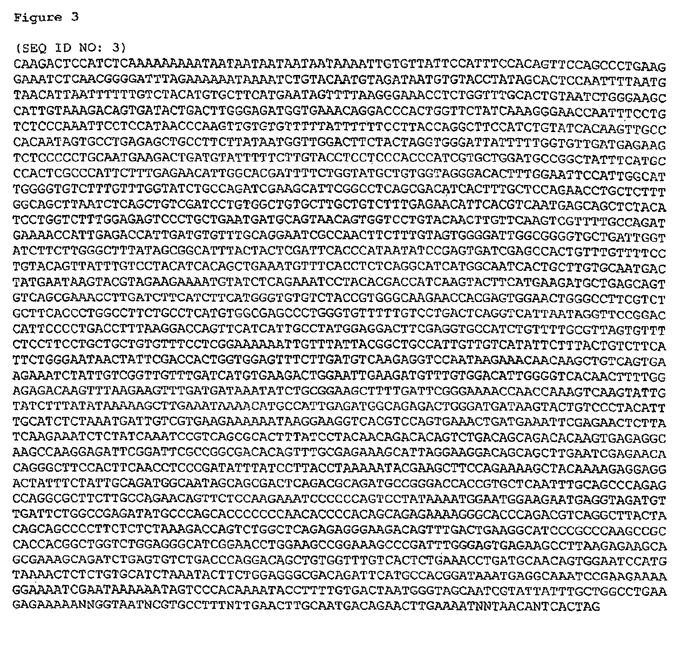

The invention also embraces the polynucleotide coding sequences that encode the human NHE2-LF and NHE2-SF polypeptides, for example, as shown in Fig. 1 (SEQ ID NO: 1) and in Fig. 3 (SEQ ID NO: 3), respectively.

As used herein, human NHE2 refers collectively to both human NHE2-LF and human NHE2-SF. The nucleic acid sequences encoding the human NHE2 may be extended utilizing a partial nucleotide sequence and employing various polymerase chain reaction (PCR)-based methods known in the art to detect upstream sequences, such as promoters and regulatory elements. For example, one method which may be employed, restriction-site PCR, uses universal and nested primers to amplify unknown sequence from genomic DNA within a cloning vector (see, e.g., Sarkar, PCR Methods Applic. 2: 318-322, 1993). Another method, inverse PCR, uses primers that extend in divergent directions to amplify unknown sequence from a circularized template. The template is derived from restriction fragments comprising a known genomic locus and surrounding sequences (see, e.g., Triglia et al., Nucleic Acids Res. 16: 8186, 1988). A third method, capture PCR, involves PCR amplification of DNA fragments adjacent to known sequences in human and yeast artificial chromosome DNA (see, e.g., Lagerstrom et al., PCR Methods Applic. 1:111-119, 1991). In this method, multiple restriction enzyme digestions and ligations may be used to insert an engineered double-stranded sequence into a region of unknown sequence before performing PCR.

In another embodiment of the invention, a polynucleotide of the invention may be cloned in recombinant DNA molecules that direct expression of the human NHE2 in appropriate host cells. The nucleotide sequences of the present invention can be engineered using methods generally known in the art in order to alter NHE2-encoding sequences for a variety of purposes including, but not limited to, modification of the cloning, processing, and/or expression of the gene product.

DNA shuffling by random fragmentation and PCR reassembly of gene fragments and synthetic oligonucleotides may be used to engineer the nucleotide sequences. For example, oligonucleotide-mediated site-directed mutagenesis may be used to introduce mutations that create new restriction sites, alter glycosylation patterns, change codon preference, produce splice variants, and so forth. In another embodiment, sequences encoding a human NHE2 may be synthesized, in whole or in part, using chemical methods well known in the art (see, e.g., Caruthers et al., Nucleic Acids Symp. Ser. 7: 215-223, 1980; and Horn et al., Nucleic Acids Symp. Ser. 7: 225-232,1980). Alternatively, the human NHE2 itself or a fragment thereof may be synthesized using chemical methods. For example, peptide synthesis can be performed using various solid-phase techniques (see, e.g., Roberge et al., Science 269: 202-204, 1995). Automated synthesis may be achieved using the ABI 431A peptide synthesizer (Perkin-Elmer, Norwalk, CT). Additionally, the amino acid sequence of human NHE2, or any part thereof, may be altered during direct synthesis and/or combined with sequences from other proteins, or any part thereof, to produce a variant polypeptide. The peptide may be substantially purified by preparative high performance liquid chromatography (see, e.g., Chiez and Regnier, Methods Enzymol. 182: 392-421, 1990). The composition of the synthetic peptides may be confirmed by amino acid analysis or by sequencing (see, e.g., Creighton, *Proteins, Structures and Molecular Properties*, WH Freeman, New York, NY 1984).

### Expression Vectors and Host Cells

In order to express a biologically active human NHE2, the nucleotide sequence encoding the human NHE2 may be inserted into an appropriate expression vector, i.e., a vector which contains the necessary elements for transcriptional and translational control of the inserted coding sequence in a suitable host. These elements include regulatory sequences, such as enhancers, constitutive and inducible promoters, and 5' and 3' untranslated regions derived from the vector and/or from the polynucleotide sequences encoding a human NHE2. Such elements may vary in their strength and specificity. Specific initiation signals may also be used to achieve more efficient translation of sequences encoding NHE2 polypeptide. Such signals include the ATG initiation codon and adjacent sequences, e.g. the Kozak sequence. In cases where sequences encoding a human NHE2 and its initiation codon and upstream regulatory sequences are inserted into the appropriate expression vector, no additional transcriptional or translational control signals may be needed. However, in cases where only coding sequence, or a fragment thereof, is inserted, exogenous translational control signals including an in-frame ATG initiation codon should be provided by the vector. Exogenous translational elements and initiation codons may be of various origins, both natural and synthetic. The efficiency of expression may be enhanced by the inclusion of enhancers appropriate for the particular host cell system used (see, e.g., Scharf et al., Results Probl. Cell Differ. 20: 125-162,1994). Methods which are well known to those skilled in the art may be used to construct expression vectors containing sequences encoding a NHE2 polypeptide and appropriate transcriptional and translational control elements. These methods include *in vitro* recombinant DNA techniques, synthetic techniques, and *in vivo* genetic recombination (see, e.g., Sambrook et al., *Molecular Cloning*, *Laboratory Manual*, Cold Spring Harbor Press, Plainview, NY, chs. 4, 8, and 16-17, 1989; Ausubel et al., *Current Protocols in Molecular Biology,* John Wiley & Sons, New York, NY, chs. 9, 13, and 16, 1995). A variety of expression vector/host systems may be utilized to contain and express sequences encoding a human NHE2. These include, but are not limited to, microorganisms such as bacteria transformed with recombinant bacteriophage, plasmid, or cosmid DNA expression vectors; yeast transformed with yeast expression vectors (e.g., episomes, chromosomal elements); insect cell systems infected with viral expression vectors (e.g., baculovirus, paponavirus, *Vaccinia*, adenovirus, pox virus, rabies virus, or retrovirus); plant cell systems transformed with viral expression vectors (e.g., cauliflower mosaic virus, CaMV, or tobacco mosaic virus, TMV), with bacterial expression vectors (e.g., Ti or pBR322 plasmids), or animal cell systems. The invention is not limited by the host cell employed.

In bacterial systems, a number of cloning and expression vectors may be selected depending upon the use intended for polynucleotide sequences encoding the human NHE2. For example, routine cloning, subcloning, and propagation of polynucleotide sequences encoding the human NHE2-LF or NHE2-SF polypeptide can be achieved using a multifunctional *E*. *coli* vector such as pBlueScript (Stratagene, La Jolla, CA) or pSport1 plasmid (Life Technologies, Gaithersburg, MD). Ligation of human NHE2-LF or NHE2-SF sequence into the vector's multiple cloning site disrupts the lacZ gene, allowing a colorimetric screening procedure for identification of transformed bacteria containing recombinant molecules. In addition, these vectors may be useful for *in vitro* transcription, dideoxy sequencing, single strand rescue with helper phage, and creation of nested deletions in the cloned sequence (see, e.g., Van Heeke and Schuster, J. Biol. Chem. 264: 5503-5509, 1989). When large quantities of NHE2 polypeptide are needed, e.g., for the production of antibodies, vectors which direct high level expression of NHE2 polypeptide may be used. For example, vectors containing the strong, inducible T5 or T7 bacteriophage promoter may be used.

Recombinant protein expression can be maximized in host bacteria by providing a genetic background wherein the host cell has an impaired capacity to proteolytically cleave the recombinant protein (Gottesman et al., Gene Expression Technology: Methods in Enzymology 185:119-28, 1990). Alternatively, the polynucleotide sequence can be altered to provide preferential codon usage for a specific host cell, e.g., *E*. *coli* (Wada et al., Nucleic Acids Res. 20: 2111-18, 1992).

In yeast expression systems, a number of vectors containing constitutive or inducible promoters, such as alpha factor, alcohol oxidase, or PGH promoters, may be used, for example, in the yeast *Saccharomyces cerevisiae* or *Pichia pastoris*. In addition, such vectors direct either the secretion or intracellular retention of expressed proteins and enable integration of foreign sequences into the host genome for stable propagation (see, e.g., Ausubel, 1995; Bitter et al., Methods Enzymol. 153: 516-544, 1987; and Scorer et al., BioTechnology 12: 181-184, 1994). Plant systems may also be used for expression of the human NHE2. Transcription of sequences encoding NHE2 polypeptide may be driven by viral promoters, e.g., the 35S and 19S promoters of CaMV used alone or in combination with the omega leader sequence from TMV (Takamatsu, EMBO J. 6: 307-311, 1987). Alternatively, plant promoters such as the small subunit of RUBISCO or heat shock promoters may be used (see, e.g., Coruzzi et al., EMBO J. 3: 1671-1680, 1984; Broglie et al., Science 224: 838-843, 1984; and Winter et al., Results Probl. Cell Differ. 17: 85-105, 1991). These constructs can be introduced into plant cells by direct DNA transformation or pathogen-mediated transfection (see, e.g., *The McGraw Hill Yearbook of Science and Technology*, McGraw Hill, New York NY, pp. 191-196, 1992).

In mammalian cells, a number of viral-based expression systems may be utilized. In cases where an adenovirus is used as an expression vector, sequences encoding NHE2 polypeptide may be ligated into an adenovirus transcription/translation complex consisting of the late promoter and tripartite leader sequence. Insertion in a non-essential E1 or E3 region of the viral genome may be used to obtain infective virus that expresses human NHE2 in infected host cells (see, e.g., Logan and Shenk, Proc. Natl. Acad. Sci. USA 81: 3655-3659, 1984). In addition, transcription enhancers, such as the Rous sarcoma virus (RSV) enhancer, may be used to increase expression in mammalian host cells. SV40 or EBV-based vectors may also be used for high-level protein expression.

HACs, BACs, or YACs may also be employed to deliver larger fragments of DNA than can be contained in and expressed from a plasmid. For example, HACs of about 6 kb to 10 Mb are constructed and delivered via conventional delivery methods (e.g., liposomes, polycationic amino polymers, or vesicles) for therapeutic purposes (see, e.g., Harrington et al., Nat. Genet. 15: 345-355, 1997). For long term production of recombinant proteins in mammalian systems, stable expression of the human NHE2 in cell lines is preferred. For example, sequences encoding the human NHE2 can be transformed into cell lines using expression vectors which may contain viral origins of replication and/or endogenous expression elements and a selectable marker gene on the same or on a separate vector. Following the introduction of the vector, cells may be allowed to grow for about 1 to 2 days in enriched media before being switched to selective media. The purpose of the selectable marker is to confer resistance to a selective agent, and its presence allows growth and recovery of cells which successfully express the introduced sequences. Resistant clones of stably transformed cells may be propagated using tissue culture techniques appropriate to the cell type.

The invention also encompasses vectors in which the nucleic acid sequences described herein are cloned into the vector in reverse orientation, but operably linked to a regulatory sequence that permits transcription of antisense RNA. Thus, an antisense transcript can be produced to all, or to a portion, of the nucleic acid molecule sequences described herein, including both coding and non-coding regions. Expression of this antisense RNA is subject to each of the parameters described above in relation to expression of the sense RNA.

Any number of selection systems may be used to recover transformed cell lines. These include, but are not limited to, the herpes simplex virus thymidine kinase and adenine phosphoribosyltransferase genes, for use in TK⁻, and APR⁻ cells, respectively (see, e.g., Wigler et al., Cell 11: 223-232, 1997; Lowy et al., Cell 22: 817-823, 1980). Also, antimetabolite, antibiotic, or herbicide resistance can be used as the basis for selection. For example, Dhfr confers resistance to methotrexate; Neo confers resistance to the aminoglycosides neomycin and G-418; and Als and Pat confer resistance to chlorsulfuron and phosphinotricin acetyltransferase, respectively (see, e.g., Wigler et al., Proc. Natl. Acad. Sci. USA 77: 3567-3570,1980; Colbere-Garapin et al., J. Mol. Biol. 150: 1-14, 1981). Additional selectable genes have been described, e.g., TrpB and HisD, which alter cellular requirements for metabolites (see, e.g., Hartman and Mulligan, Proc. Natl. Acad. Sci. USA 85: 8047-8051, 1988). Visible markers, e.g., anthocyanins, green fluorescent proteins (GFP; Clontech, Palo Alto, CA), β-glucuronidase and its substrate β-glucuronide, or luciferase and its substrate luciferin may be used. These markers can be used not only to identify transformants, but also to quantify the amount of transient or stable protein expression attributable to a specific vector system (see, e.g., Rhodes, Methods Mol. Biol. 55: 121-131, 1995). Although the presence/absence of marker gene expression suggests that the gene of interest is also present, the presence and expression of the gene may need to be confirmed. For example, if the sequence encoding the human NHE2 is inserted within a marker gene sequence, transformed cells containing sequences encoding NHE2 polypeptide can be identified by the absence of marker gene function. Alternatively, a marker gene can be placed in tandem with a sequence encoding NHE2 polypeptide under the control of a single promoter. Expression of the marker gene in response to induction or selection usually indicates expression of the tandem gene as well.

In general, host cells that contain the nucleic acid sequence encoding the human NHE2 and that express the human NHE2 may be identified by a variety of procedures known to those of skill in the art. These procedures include, but are not limited to, DNA-DNA or DNA-RNA hybridizations, PCR amplification, and protein bioassay or immunoassay techniques which include membrane, solution, or chip based technologies for the detection and/or quantification of nucleic acid or protein sequences.

Immunological methods for detecting and measuring the expression of NHE2 using either specific polyclonal or monoclonal antibodies are known in the art in light of this disclosure. Examples of such techniques include enzyme-linked immunosorbent assays (ELISAs), radioimmunoassays (RIAs), Western blots, immunoprecipitation, immunofluorescence, and fluorescence activated cell sorting (FACS). These and other assays are well known in the art (see, e.g., Hampton, *Serological Methods*, *A Laboratory Manual,* APS Press, St. Paul, MN, Sect. IV, 1990; Coligan et al., *Current Protocols in Immunology*, Greene Pub. Associates and Wiley-lnterscience, New York NY, 1997; and Pound, *Immunochemical Protocols,* Humana Press, Totowa, NJ, 1998). A wide variety of labels and conjugation techniques are known by those skilled in the art and may be used in various nucleic acid and amino acid assays.

Means for producing labelled hybridization or PCR probes for detecting sequences related to polynucleotides encoding the human NHE2 include oligolabelling, nick translation, end-labelling, or PCR amplification using a labelled nucleotide.

Alternatively, the sequences encoding the human NHE2, or any fragments thereof, may be cloned into a vector for the production of an mRNA probe. Such vectors are known in the art, are commercially available, and may be used to synthesize RNA probes *in vitro* by addition of an appropriate RNA polymerase such as T7, T3, or SP6 and labelled nucleotides. These procedures may be conducted using a variety of commercially available kits (e.g., Amersham Pharmacia Biotech, Piscataway, NJ; Promega, Madison, Wl; and US Biochemical, Cleveland, OH). Suitable reporter molecules or labels which may be used for ease of detection include radionuclides, enzymes, fluorescent, chemiluminescent, or chromogenic agents, as well as substrates, cofactors, inhibitors, magnetic particles, and the like.

Host cells transformed with nucleotide sequences encoding NHE2 polypeptide may be cultured under conditions suitable for the expression and recovery of the protein from cell culture. The protein produced by a transformed cell may be secreted or retained intracellularly depending on the sequence and/or the vector used. As will be understood by those of skill in the art, expression vectors containing polynucleotides which encode the human NHE2 may be designed to contain signal sequences which direct secretion of the human NHE2 through a prokaryotic or eukaryotic cell membrane. In addition, a host cell strain may be chosen for its ability to modulate expression of the inserted sequences or to process the expressed protein in the desired fashion. Such modifications of the polypeptide include, but are not limited to, acetylation, carboxylation, glycosylation, phosphorylation, lipidation, and acylation. Post-translational processing which cleaves a "prepro" or "pro" form of the protein may also be used to specify protein targeting, folding, and/or activity.

Different host cells which have specific cellular machinery and characteristic mechanisms for post-translational activities (e.g., CHO, HeLa, MDCK, HEK293, and W138) are available from the American Type Culture Collection (ATCC, Manassas, VA) and may be chosen to ensure the correct modification and processing of the foreign protein. Preferably, the host cell is deficient in endogenous Na⁺/H⁺ exchange activity, for example, PS120 cells (Pouyssegur et al., Proc. Natl. Acad. Sci. USA 81: 4833-37, 1984). In another embodiment of the invention, natural, modified, or recombinant nucleic acid sequences encoding NHE2 polypeptide may be ligated to a heterologous sequence resulting in translation of a fusion protein in any of the aforementioned host systems. For example, a chimeric NHE2 protein containing a heterologous moiety that can be recognized by a commercially available antibody may facilitate the screening of peptide libraries for modulators of NHE2 polypeptide activity. Heterologous protein and peptide moieties may also facilitate purification of fusion proteins using commercially available affinity matrices. Such moieties include, but are not limited to, glutathione S-transferase (GST), maltose binding protein (MBP), thioredoxin (Trx), calmodulin binding peptide (CBP), 6-His, FLAG, c-myc, and hemagglutinin (HA). GST, MBP, Trx, CBP, and 6-His enable purification of their cognate fusion proteins on immobilized glutathione, maltose, phenylarsine oxide, calmodulin, and metal-chelate resins, respectively. FLAG, c-myc, and hemagglutinin (HA) enable immunoaffinity purification of fusion proteins using commercially available monoclonal and polyclonal antibodies that specifically recognize these epitope tags. A fusion protein may also be engineered to contain a proteolytic cleavage site located between the NHE2 polypeptide encoding sequence and the heterologous protein sequence, so that NHE2 polypeptide may be cleaved away from the heterologous moiety following purification. Methods for fusion protein expression and purification are discussed in Ausubel (1995, *supra*, ch. 10). A variety of commercially available kits may also be used to facilitate expression and purification of fusion proteins.

In a further embodiment of the invention, synthesis of a radiolabelled human NHE2 may be achieved *in vitro* using the TNT rabbit reticulocyte lysate or wheat germ extract system (Promega). These systems couple transcription and translation of protein-coding sequences operably associated with the T7, T3, or SP6 promoters. Translation takes place in the presence of a radiolabelled amino acid, for example, ³⁵S-methionine.

Fragments of the human NHE2 may be produced not only by recombinant means, but also by direct peptide synthesis using solid-phase techniques (see, e.g., Creighton, *supra*, pp. 55-60). Protein synthesis may be performed by manual techniques or by automation. Automated synthesis may be achieved, for example, using the ABI® 431A peptide synthesizer (Perkin-Elmer). Various fragments of the human NHE2 may be synthesized separately and then combined to produce the full length molecule.

### Cell Based Screening Assays

Host cells of the invention, expressing either the human NHE2-LF or human NHE2-SF, can be used in assays to measure NHE2-mediated Na⁺/H⁺ exchange. Preferably, the host cells are deficient in endogenous Na⁺/H⁺ exchange, e.g., PS120 cells (Pouyssegur et al., Proc. Natl. Acad. Sci. USA 81:4833-37, 1984).

The functional expression of human NHE2-LF or NHE-SF in host cells can be confirmed by any standard method known in the art, including acidic loading, as further described in Example 3 below. Test agents or controls can be incubated with the cells and tested for their ability to modulate Na⁺/H⁺ exchange using a standard method, e.g., using fluorescent laser imaging plate reader (FLIPR) technology or assessing ²²Na⁺ transport, as described in Examples 4 and 5, respectively, and in the art (Collins et al., Proc. Natl. Acad. Sci. USA 90: 3938-42, 1993, Brant et al., WO 97/03196).

Such assays are useful to identify agents that modulate NHE2 transport activity. Alternatively, such host cells can be used as negative selection controls when screening for agents that modulate another NHE (e.g., NHE1 or NHE3) selectively without significantly affecting NHE2-mediated transport. For example, given that both NHE2-LF and NHE2-SF are expressed in the intestine (see Example 2), selectivity of a compound against this isoform may be important for an oral therapeutic that is targeted to inhibit another NHE isoform.

### Nucleic Acid Arrays

The present invention further provides nucleic acid detection kits, such as arrays or microarrays of nucleic acid molecules that are based on the sequence information provided in Fig. 1 and Fig. 3.

As used herein arrays or microarrays refer to an array of distinct polynucleotides or oligonucleotides synthesized on a substrate, such as paper, nylon, or other type of membrane, filter, chip, glass slide, or any other suitable solid support. In one embodiment, the microarray is prepared and used according to methods described in Chee et al., U.S. Pat. No. 5,837,832, Chee et al., WO 95/11995, Lockhart et al., Nat. Biotech. 14: 1675-80, 1996, Schena et al., Proc. Natl. Acad. Sci. 93: 10614-19,1996. Other arrays are produced by the methods described in Brown et al., U.S. Pat. No. 5,807,522, and in Baldeschwieler et al., WO 95/25116.

### Example 1. Cloning Human NHE-2

Unless otherwise noted, restriction endonucleases and other biological reagents were obtained from New England Biolabs (Beverly, MA).

Initially, a reverse transcription polymerase chain reaction (RT-PCR) focused approach was attempted to clone human NHE2 based upon a published sequence reported to be human NHE2 (Ghishan et al., Genomics 30: 25-30, 1995). Although correct size PCR fragments were isolated when utilizing rat mRNA as a template, the result was not repeated using human mRNA. Thus, it was concluded that the sequence reported in Ghishan et al., *supra*, was rat and not human. A partial human NHE2 sequence was later reported by Dudeja et al., Am. J. Physiol. 271: G483-G493, 1996 (see also Genbank S83549). This sequence was used to isolate a human NHE-2 cDNA fragment from human small intestine mRNA.

The 3' end of the full length human NHE-2 cDNA was cloned using RT-PCR, human small intestine mRNA, and Superscript™ Reverse Transcriptase (RT) (Gibco BRL, Grand Island, NY). PCR was then performed using the GC-advantage cDNA kit (Clontech). The 5' and 3' primers used were P1 and P2, respectively (all primers used for PCR reactions are described in Table 1). P1 corresponds to nucleotides 351-382 of the partial human sequence of Genbank S83549 and P2 corresponds to nucleotides 2933-2912 of the rat sequence of Genbank L11236. Cycling conditions included 35 cycles at 94°C for 30 sec., 60°C for 30 sec., and 72°C for 3 min.

For 5' RACE, the mRNA was primed with random hexamers, and reversed transcribed using Superscript™ RT. The reverse transcribed cDNA was tailed using Terminal Transferase (Boehringer Mannheim, Indianapolis, IN) and dATP. The 5' end of short form human NHE2 clone (NHE2-SF) was obtained by two independent PCR reactions of 35 cycles each using the above-described cycling conditions. The first reaction used P3 (nucleotides 1030-1059 of Genbank L11236) as the 5' primer and P4 (corresponding to nucleotides 1973-1943 of the long form human NHE2 (NHE2-LF)) as the 3' primer. The second reaction used P5 (the reverse primer of P3) as the 3' primer and P6 (nucleotides 501-520 of Genbank L11236) as the 5' primer.

The complete 5' end of NHE2-LF was amplified using Marathon-Ready™ small intestine cDNA (Clontech, Palo Alto, CA) and the GC-advantage cDNA kit using 10% GC-Melt for 35 cycles at 94°C for 30 sec., 60°C for 30 sec., and 68°C for 3 min. The first PCR reaction used AP1 (Clontech) and P8 as 5' and 3' primers, respectively. The second PCR reaction used 5' primer AP2 (Clontech) and 3' primer P9 (corresponding to nucleotides 329-295 of the human NHE2-LF).

To complete the 5' end of NHE2-SF, the PCR amplification was performed in 35 cycles at 94°C for 30 sec., 60°C for 30 sec., and 72°C for 3 min. The first reaction used a 5' anchor primer (Ro-dT17) and 3' primer P7 (nucleotides 520-498 of the human NHE2-LF). This was followed by nested PCR with Ro-dT17a (primer within Ro-dT17) and P8 (nucleotides 492-465 of the human NHE2-LF) as the 5' and 3' primers, respectively.

The resultant PCR fragments were subcloned and assembled into a pCRII TA cloning vector (Invitrogen, Carlsbad, CA). Three independent clones were sequenced with 2-4 overlapping sequence passes on both complementary DNA strands. The full length NHE2-LF and NHE2-SF sequences were assembled into pCRII by PCR extension. Notl/Bglll fragments including each of the entire coding regions were subcloned into pcDNA3.1 (-) vectors (Invitrogen) digested with Notl/BamHl.

Similar to the rat NHE2 sequence, the human NHE2 polynucleotide sequence was found to contain two potential open reading frames (Met1 and Met 117, as shown in Fig. 2). To determine which initiation codon of the transcript is used for translation, an *in vitro* S³⁵ labeling transcription/translation assay using the T7 promoter in PCDNA3.1 containing the NHE-LF or NHE-SF sequence was used to assess transcription. An assessment of labeled protein bands revealed that two major bands close to the predicted size of both the NHE2-LF (115 kD) and NHE2-SF (89 kD) were expressed.

**Table 1.**

| **Primer Sequences** | | |
|---|---|---|
| Primer | Sequence (5'-3') | |
| P1 | AGACACAAGTGAGAGGCAAGCCAAGGAGATTC | SEQ ID NO:5 |
| P2 | TTAAATTTTCAAGTTCTGT | SEQ ID NO:6 |
| P3 | GTGGGGATTGGCGGGGTGCTGATTGGTATC | SEQ ID NO:7 |
| P4 | AAAGTGCGCTGACGGATTTGATAGAGATTTC | SEQ ID NO:8 |
| P5 | GATACCAATCAGCACCCCGCCAATCCCCAC | SEQ ID NO:9 |
| P6 | GTGCCGGAGAGCTGTCTTCT | SEQ ID NO:10 |
| RodT17 | AAGCATCCGTCAGCATCGGCAGGACAACTTTTTTTT TTTTTTTTT | SEQ ID NO:11 |
| RodT17a | AGCATCGGCAGGACAAC | SEQ ID NO:12 |
| P7 | GAGACTTCTCAACACCAAA | SEQ ID NO:13 |
| P8 | CCCACCTAGTAGAAGTCCAACCATTATA | SEQ ID NO:14 |
| AP1 | GTAATACGACTCACTATAGGGC | SEQ ID NO:15 |
| AP2 | ACTATAGGGCACGCGTGGT | SEQ ID NO:16 |
| P9 | GGCAGCCGGCTCTCCTCGAACAGCGTCGTTCCGGG | SEQ ID NO:17 |

### Example 2. NHE2 Expression Patterns

RT-PCR and probes specific for NHE2-LF and NHE2-SF were used to determine the tissue expression patterns for human NHE2-LF and NHE2-SF. The results of 30 cycles of competitive PCR amplification indicated that NHE2-LF is the predominant form expressed in small intestine, stomach, and skeletal muscle, although lower levels of NHE2-SF were expressed in these tissues. Conversely, NHE2-SF was the predominant form expressed in kidney and placenta. NHE2-SF was the predominant form expressed in the heart.

### Example 3. Selection of NHE2-LF and NHE2-SF Expressing Cells by Acidic Loading

The two alternative cDNA forms of human NHE2, NHE2-LF and NHE2-SF, were stably expressed in the NHE-deficient cell line PS120 (obtained from Dr. Pouyssegur, Centre de Biochimie, CNRS, Universite de Nice, Nice, France, see also Pouyssegur et al., Proc. Natl. Acad. Sci. USA 81:4833-37, 1984). The pCDNA3.1 vectors containing human NHE2-LF and NHE2-SF coding sequences were transfected into separate samples of PS120 cells using the FuGENE6™ (Boehringer Mannheim) transfection agent method. Briefly, PS120 cells were cultured in T175 flasks to 60-80% confluency. Following a 15 min. incubation 20 µg of DNA with FuGENE6™ suspended in 100 µl OptiMem medium (Gibco BRL), the DNA-lipid complex was suspended in 30 ml DMEM containing 10% fetal bovine serum, applied to the cells, and incubated overnight. Cells were selected using G418 and maintained in culture using DMEM with 500 µg/ml G418.

Recombinant NHE2 expression in these lines was verified by the selection technique of acidic loading, preferably performed on a weekly to monthly basis. The growth medium was aspirated from flasks containing NHE2 transfected PS120 cells, and the cells were incubated with 25 ml of loading medium (70 mM choline chloride, 50 mM NH₄Cl, 5 mM KCI, 1 mM MgCl_{2,} 1.8 mM CaCl₂, 5 mM glucose, 15 mM Hepes acid, pH 7.5 (pH adjusted with 1M Tris base)) for 1 hour in a 37°C humidified incubator lacking CO₂.

The loading medium was aspirated from the cells, which were then washed quickly with 25 ml of wash medium (120 mM choline chloride, 5 mM KCI, 1 mM MgCl₂, 1.8 mM CaCl₂, 5 mM glucose, 15 mM MOPS, pH 7.0 (pH adjusted with 1M Tris base). The wash medium was aspirated and 25 ml of recovery medium (120 mM NaCI, 5 mM KCI, 1 mM MgCl_{2,} 1.8 mM CaCl_{2,} 5 mM glucose, 15 mM MOPS, pH 7.0 (pH adjusted with 1M Tris base)) was added. The cells were incubated for 1 hour in a 37°C humidified incubator lacking CO₂. As a final step, the recovery medium was aspirated, replaced with 25 ml growth medium (DMEM high glucose with L-glutamine, pyridoxine HCI, and lacking sodium pyruvate (Gibco, Cat. No. 11965-092), with 10% heat inactivated fetal bovine serum (Gibco, Cat. No. 10082-147), 0.5% 10 U/ml penicillin G sodium/10 µg/ml streptomycin sulfate (Gibco BRL, Cat. No. 15140-122). The cells were returned to a 37°C 5% CO₂ humidified incubator. Both PS120 cells expressing NHE2-LF and NHE2-SF survived acidic loading, indicating that both forms of NHE2 polypeptide were functional NHEs. Cells lines surviving this procedure were subjected to further functional assays, as further described below.

### Example 4. Functional Activity Assessed by FLIPR Assay

Human NHE2-LF and human NHE2-SF transfected PS120 cells were grown to confluence in collagen coated Fluorescent Laser Imaging Plate Reader (FLIPR) plates. To remove growth medium, the cells were washed with a 10 mM Hepes buffer, pH 7.4. A pH sensitive fluorescent dye, BCECF, AM (5 µM) (Molecular Probes, Eugene, OR), was added to each well and the plates were incubated in a 37°C humidified 5% CO₂ incubator for approximately 20 minutes.

The cells were then washed with 30 mM NH₄Cl buffer, pH 7.5, to remove excess dye. Plates were incubated with this buffer for approximately 45 minutes in a 37°C humidified incubator.

5-(N,N-Hexamethylene)-amiloride ((hexa)-amiloride), amiloride, or a vehicle, was then added to each well and allowed to incubate for a minimum of 5 minutes prior to the initiation of intracellular acidification. The NH₄Cl buffer was removed and the plates were placed on the FLIPR to establish a baseline fluorescence. A 10 mM hepes buffer, pH 7.4, containing the appropriate concentration of test compound or vehicle was added to each well of the plate. (This process initiates intracellular acidification and the quenching of BCECF-related fluorescence. The resultant activation of the Na⁺/H⁺ exchanger and the decrease in intracellular H⁺ ions produces in gradual increase in dye fluorescence towards baseline.) Changes in fluorescence were measured by the FLIPR over a period of 7 min. Activity of the exchanger was calculated based on the percentage of fluorescence recovery as compared to control in the presence of varying concentrations of compounds (Table 2).

**Table 2.**

| **IC50 Values Determined By FLIPR** | | | |
|---|---|---|---|
| Compound | NHE2-LF | NHE2-SF line #1 | NHE2-SF line #2 |
| (hexa)-Amiloride | 7.48 µM | 23 nM | 45 nM |
| Amiloride | 27 µM | 1.7 µM | 1.37 µM |

### Example 5. Functional Activity as Assessed by ²²Na⁺ Assay

NHE transfected PS120 cells were plated into collagen-coated 24 well plates and grown to confluence in growth medium (*supra*). The medium was removed from the plates and 30 mM NH₄Cl buffer, pH 7.5, was added to each well for approximately 45 min.

Wells were quickly washed with a choline chloride buffer devoid of sodium chloride. A sodium chloride free buffer (0.2 µCi/ml of ²²Na⁺; 1 mM ouabain; test compound, at varying concentrations, or vehicle, or 100 µM (hexa)-amiloride was then added to each of the appropriate wells of the plate.

Plates were incubated with this buffer for 6 minutes, the reaction mix was aspirated, and the reaction was terminated by washing the cell monolayer three times with 0.1 M of ice cold MgCl₂. After solubilization with 0.1 N NaOH, aliquots were taken from each well, transferred to vials containing scintillation cocktail, and counted for 2 min. on a liquid scintillation counter.

Inhibition of the Na⁺/H⁺ exchanger was calculated as % of total counts minus nonspecific counts in wells with test compound as compared to control wells.

## Claims

1. An isolated and/or purified polypeptide comprising the amino acid sequence of SEQ ID NO: 2 or 4.

2. An isolated or purified polypeptide comprising:
(a) an amino acid sequence encoded by a polynucleotide which will hybridize under highly stringent conditions with the complement of the coding sequence shown in SEQ ID NO: 1 or 3; or
(b) an amino acid sequence having at least 95% identity to an amino acid sequence comprising SEQ ID NO: 2 or 4;
wherein said polypeptide has Na⁺/H⁺ exchange (NHE) activity.

3. An isolated or purified polynucleotide comprising:
(a) a nucleic acid sequence encoding a polypeptide of claim 1; or
(b) the coding sequence of SEQ ID NO: 1 or 3.

4. An isolated and/or purified polynucleotide comprising a nucleic acid sequence which hybridises with the complement of the full length coding sequence of SEQ ID NO: 1 or 3 under highly stringent conditions, wherein said polynucleotide encodes a polypeptide with NHE activity.

5. An antibody that selectively binds to a polypeptide of claim 1 or 2.

6. A vector comprising a polynucleotide of claim 3 or 4.

7. A host expressing a heterologous protein of claim 1 or 2.

8. A cell membrane preparation from the host of claim 7.

9. A method of screening for an agent that modulates human NHE2 activity, said method comprising contacting an agent with a host cell that expresses a heterologous NHE2 comprising SEQ ID NO: 2 or 4, and measuring NHE activity in said cell, wherein a difference between said NHE activity in the presence of the agent and in the absence of the agent is indicative that the agent modulates said activity.

10. The method of claim 9, wherein said host cell lacks endogenous NHE activity.

11. The method of claim 9, wherein said host cell is a PS120 cell.

12. An agent as identified by the method of any one of claims 9 to 11.
